# EUROPEAN PATENT SPECIFICATION

(11) **EP 1 600 507 B1**
(45) Date of publication and mention of the grant of the patent: **19.01.2011**
(21) Application number: 04712761.8
(22) Date of filing: 19.02.2004
(51) Int. Cl.: C12N 15/09, A01K 67/027, A61K 38/00, A61K 39/395, A61K 45/00, C07K 14/47, C12Q 1/02, C12Q 1/68, G01N 33/15, G01N 33/50

(54) **Mutant SREBP-1c promoters and use thereof**
Mutierte SREBP-1c Promoteren und deren Verwendung
Promoteurs SREBP-1c mutants et leur utilisation

(30) Priority: 20.02.2003 JP 2003043398; 05.06.2003 JP 2003160856
(43) Date of publication of application: 30.11.2005
(73) Proprietor: Takeda Pharmaceutical Company Limited, Osaka-shi, Osaka 541-0045 (JP)
(72) Inventor: NISHIO, Yoshihiko, 5200818 (JP); MAEGAWA, Hiroshi, 5250000 (JP); KASHIWAGI, Atsunori, 5202264 (JP)
(74) Representative: Wright, Robert Gordon McRae
(86) International application number: PCT/JP2004/001924
(87) International publication number: WO 2004/074475

(56) References cited:
- AMEMIYA-KUDO M ET AL: "Promoter analysis of the mouse sterol regulatory element-binding protein-1c gene" JOURNAL OF BIOLOGICAL CHEMISTRY, AMERICAN SOCIETY OF BIOLOCHEMICAL BIOLOGISTS, BIRMINGHAM,, US, vol. 275, no. 40, 6 October 2000 (2000-10-06), pages 31078-31085, XP002979622 ISSN: 0021-9258
- DENG XIONG ET AL: "Regulation of the rat SREBP-1c promoter in primary rat hepatocytes" BIOCHEMICAL AND BIOPHYSICAL RESEARCH COMMUNICATIONS, vol. 290, no. 1, 11 January 2002 (2002-01-11), pages 256-262, XP002383774 ISSN: 0006-291X
- NAGAI Y ET AL: "Amelioration of high fructose-induced metabolic derangements by activation of PPAR[alpha]" AMERICAN JOURNAL OF PHYSIOLOGY - ENDOCRINOLOGY AND METABOLISM 2002 UNITED STATES, vol. 282, no. 5 45-5, 2002, pages E1180-E1190, XP002383775 ISSN: 0193-1849
- AMEMIYA-KUDO M. ET AL.: 'Promoter analysis of the mouse sterol regulatory element-binding protein-1c gene' J. BIOL. CHEM. vol. 275, no. 40, 06 October 2000, pages 31078 - 31085, XP002979622
- REPA J.J. ET AL.: 'Regulation of mouse sterol regulatory element-binding protein-1c gene (SREBP-1c), by oxysterol receptors, LXRalpha and LXRbeta' GENES DEV. vol. 14, no. 22, 15 November 2000, pages 2819 - 2830, XP000961212
- SHIMANO H.: 'Sterol regulatory element-binding protein-1 as a dominant transcription factor for gene regulation of lipogenic enzymes in the liver' TRENDS CARDIOVASC. MED. vol. 10, no. 7, October 2000, pages 275 - 278, XP002979623
- OSADA RYOKO ET AL.: 'Kokatoshoku fuka ni taisuru kessei shishitsu to kan SREBP-1,PPAR-alpha han'no no mouse-keitokansa no kento' TONYOBYO vol. 45, no. SUPP2, 2002, page S-247, XP002904670
- HALLIGAN B.D. ET AL.: 'Cloning of the murine cDNA encoding VDJP, a protein homologous to the large submit of replication factor C and bacterial DNA ligases' GENE vol. 161, no. 2, 19 August 1995, pages 217 - 222, XP004042086
- STRAUSBERG R.L. ET AL.: 'Generation and initial analysis of more than 15 000 full-length human and mouse cDNA sequences' PROC. NATL. ACAD. SCI. USA vol. 99, no. 26, 24 December 2002, USA, pages 16899 - 16903, XP002964739
- DELBRIDGE M.L. ET AL.: 'Evolution of mammalian HNRPG and its relationship with the putative ezoospermia factor RBM' MAMM. GENOME. vol. 9, no. 2, February 1998, pages 168 - 170, XP002979624
- SHIMANO H. ET AL.: 'Sterol regulatory element-binding proteins (SREBPs):transcriptional regulators of lipid synthetic genes' PROG. LIPID. RES. vol. 40, no. 6, November 2001, pages 439 - 452, XP002979625
- SHIMIZU S. ET AL.: 'Protein-tyrosine phosphatase 1B as new activator for hepatic lipogenesis via sterol regulatory element-binding protein-1 gene expression' J. BIOL. CHEM. vol. 278, no. 44, 31 October 2003, pages 43095 - 43101, XP002979626

### SEQUENCE LISTING

<110> Takeda Chemical Industries, Ltd.
<120> cis-element regulating transcription, transcriptional regulatory factor binding specifically thereto and use of the same
<130> 3145WO0P
<150> JP 2003-043398
   <151> 2003-02-20
<150> JP 2003-160856
   <151> 2003-06-05
<160> 13
<170> PatentIn version 3.2
<210> 1
   <211> 574
   <212> DNA
   <213> Mus musculus
<220>
   <221> source
   <223> /note="CBA mouse"
<220>
   <221> promoter
   <222> (1)..(574)
<220>
   <221> TATA_signal
   <222> (545)..(550)
<400> 1
   ggatccagaa ctggatcatc agcccccccc tccttgaaac aagtgttctc atcctggggc      60
   gctctgctag ctagatgacc ctgcaccacc aactgccact atctaaaggc agctattggc     120
   cttcctcaga ctgtaggcaa atcttgctgc tgccattcga tgcgaagggc caggagtggg     180
   taaactgagg ctaaaatggt ccaggcaagt tctgggtgtg tgcgaacgaa ccagcggtgg     240
   gaacacagag cttccgggat caaagccaga cgccgtccgg attccggacc caggctcttt     300
   tcggggatgg ttgcctgtgc ggcaggggtt gggacgacag tgaccgccag taaccccagc     360
   gcgcgctggc gcagacgcgg ttaaaggcgg acgcccgcta gtaaccccgg ccccattcag     420
   agcaccggga gaaacccgag ctgccgccgt cgggggtggg cggggcccta atggggcgcg     480
   gcgcggctgc tgattggcca tgtgcgctca cccgaggggc ggggcacgga ggcgatcggc     540
   gggctttaaa gcctcgcggg gcctgacagg tgaa                                 574
<210> 2
   <211> 1284
   <212> DNA
   <213> Mus musculus
<220>
   <221> CDS
   <222> (1)..(1284)
<400> 2
   atg gtg gag aac agg atc atc tat gac tca gac tca gag tca gag gag 48
   Met Val Glu Asn Arg Ile Ile Tyr Asp Ser Asp Ser Glu Ser Glu Glu
   1 5 10 15
   aca gta cag gta aaa aat gct aaa aag aaa tca gaa aaa ttg tca ctg 96
   Thr Val Gln Val Lys Asn Ala Lys Lys Lys Ser Glu Lys Leu Ser Leu
   20 25 30
   tct tat aaa cct ggt aaa gtc tct cag aag gat cct gtc acc tac gtc 144
   Ser Tyr Lys Pro Gly Lys Val Ser Gln Lys Asp Pro Val Thr Tyr Val
   35 40 45
   tct gag aca gat gaa gat gat gac ttt gta tgt aaa aag gca gcc tcc 192
   Ser Glu Thr Asp Glu Asp Asp Asp Phe Val Cys Lys Lys Ala Ala Ser
   50 55 60
   aaa tca aaa gag aat gga gta tct aca aat agt tac ctt gga aca tca 240
   Lys Ser Lys Glu Asn Gly Val Ser Thr Asn Ser Tyr Leu Gly Thr Ser
   65 70 75 80
   aac gtg aaa aaa aat gaa gaa aac gtt aag act aag aac aag ccg tta 288
   Asn Val Lys Lys Asn Glu Glu Asn Val Lys Thr Lys Asn Lys Pro Leu
   85 90 95
   tct cca ata aaa ctc aca cca acg tca gtg ctc gac tat ttt gga act 336
   Ser Pro Ile Lys Leu Thr Pro Thr Ser Val Leu Asp Tyr Phe Gly Thr
   100 105 110
   gaa agt gtc cag aga tct ggg aag aag atg gtg aca agc aaa agg aaa 384
   Glu Ser Val Gln Arg Ser Gly Lys Lys Met Val Thr Ser Lys Arg Lys
   115 120 125
   gaa tct tct caa aac aca gag gat tcc aga tta aat gat gag gcc atc 432
   Glu Ser Ser Gln Asn Thr Glu Asp Ser Arg Leu Asn Asp Glu Ala Ile
   130 135 140
   gcc aag cag ctg cag ctg gat gaa gat gca gag ctg gag agg cag ttg 480
   Ala Lys Gln Leu Gln Leu Asp Glu Asp Ala Glu Leu Glu Arg Gln Leu
   145 150 155 160
   cat gaa gat gaa gaa ttt gca aga aca ctg gcc tta ttg gat gaa gaa 528
   His Glu Asp Glu Glu Phe Ala Arg Thr Leu Ala Leu Leu Asp Glu Glu
   165 170 175
   ccc aag atc aaa aag gcc cga aag gat tct gaa gag gga gaa gaa tca 576
   Pro Lys Ile Lys Lys Ala Arg Lys Asp Ser Glu Glu Gly Glu Glu Ser
   180 185 190
   ttt tca tct gtc caa gat gat tta agc aaa gca gaa aag cag aaa agc 624
   Phe Ser Ser Val Gln Asp Asp Leu Ser Lys Ala Glu Lys Gln Lys Ser
   195 200 205
   cct aat aaa gcc gag ctt ttc tca act gca aga aag acc tac agt cct 672
   Pro Asn Lys Ala Glu Leu Phe Ser Thr Ala Arg Lys Thr Tyr Ser Pro
   210 215 220
   gct aag cat ggc aag ggg agg gct tca gaa gat gct aag cag cct tgc 720
   Ala Lys His Gly Lys Gly Arg Ala Ser Glu Asp Ala Lys Gln Pro Cys
   225 230 235 240
   aaa tca gct cac cgg aag gaa gcc tgt tcc tca ccc aag gcc agt gcc 768
   Lys Ser Ala His Arg Lys Glu Ala Cys Ser Ser Pro Lys Ala Ser Ala
   245 250 255
   aaa ctg gcg ctt atg aaa gca aaa gaa gaa agt tct tac aac gaa aca 816
   Lys Leu Ala Leu Met Lys Ala Lys Glu Glu Ser Ser Tyr Asn Glu Thr
                   260                      265                      270
   gag ctg ctg gct gca aga aga aaa gaa agc gcc act gaa ccc aaa gga       864
   Glu Leu Leu Ala Ala Arg Arg Lys Glu Ser Ala Thr Glu Pro Lys Gly
           275                 280                 285
   gag aaa aca act cct aag aaa acg aaa gtt tct cca act aag aga gag       912
   Glu Lys Thr Thr Pro Lys Lys Thr Lys Val Ser Pro Thr Lys Arg Glu
       290                 295                 300
   tct gta agc cca gaa gac tct gaa aag aag cgc act aat tat caa gct       960
   Ser Val Ser Pro Glu Asp Ser Glu Lys Lys Arg Thr Asn Tyr Gln Ala
   305                 310                 315                 320
   tat cga agc tac ttg aac cga gaa ggt ccc aaa gct ctg ggc tcc aaa      1008
   Tyr Arg Ser Tyr Leu Asn Arg Glu Gly Pro Lys Ala Leu Gly Ser Lys
                   325                 330                 335
   gaa ata ccc aag gga gct gaa aat tgc ttg gaa ggc ctg acg ttt gtg      1056
   Glu Ile Pro Lys Gly Ala Glu Asn Cys Leu Glu Gly Leu Thr Phe Val
               340                 345                 350
   atc aca gga gtg ctg gag tcc att gaa cga gac gaa gcc aag tct cta      1104
   Ile Thr Gly Val Leu Glu Ser Ile Glu Arg Asp Glu Ala Lys Ser Leu
           355                 360                 365
   att gaa cgt tat ggg ggg aaa gta aca gga aac gtg agc aag aaa acc      1152
   Ile Glu Arg Tyr Gly Gly Lys Val Thr Gly Asn Val Ser Lys Lys Thr
       370                 375                 380
   aac tac ctc gtc atg ggc cgc gac agt ggg cag tcc aag agt gac aag      1200
   Asn Tyr Leu Val Met Gly Arg Asp Ser Gly Gln Ser Lys Ser Asp Lys
   385                 390                 395                 400
   gca gca gct ctg gga aca aaa atc ctc gat gaa gac ggc ctg ttg gat      1248
   Ala Ala Ala Leu Gly Thr Lys Ile Leu Asp Glu Asp Gly Leu Leu Asp
                   405                 410                 415
   ctg att cga act atg cca ggc aag aga tcc aag tac                      1284
   Leu Ile Arg Thr Met Pro Gly Lys Arg Ser Lys Tyr
               420                 425
<210> 3
   <211> 428
   <212> PRT
   <213> Mus musculus
<400> 3
   Met Val Glu Asn Arg Ile Ile Tyr Asp Ser Asp Ser Glu Ser Glu Glu
   1 5 10 15
   Thr Val Gln Val Lys Asn Ala Lys Lys Lys Ser Glu Lys Leu Ser Leu
   20 25 30
   Ser Tyr Lys Pro Gly Lys Val Ser Gln Lys Asp Pro Val Thr Tyr Val
   35 40 45
   Ser Glu Thr Asp Glu Asp Asp Asp Phe Val Cys Lys Lys Ala Ala Ser
   50 55 60
   Lys Ser Lys Glu Asn Gly Val Ser Thr Asn Ser Tyr Leu Gly Thr Ser
   65 70 75 80
   Asn Val Lys Lys Asn Glu Glu Asn Val Lys Thr Lys Asn Lys Pro Leu
   85 90 95
   Ser Pro Ile Lys Leu Thr Pro Thr Ser Val Leu Asp Tyr Phe Gly Thr
   100 105 110
   Glu Ser Val Gln Arg Ser Gly Lys Lys Met Val Thr Ser Lys Arg Lys
   115 120 125
   Glu Ser Ser Gln Asn Thr Glu Asp Ser Arg Leu Asn Asp Glu Ala Ile
   130 135 140
   Ala Lys Gln Leu Gln Leu Asp Glu Asp Ala Glu Leu Glu Arg Gln Leu
   145 150 155 160
   His Glu Asp Glu Glu Phe Ala Arg Thr Leu Ala Leu Leu Asp Glu Glu
   165 170 175
   Pro Lys Ile Lys Lys Ala Arg Lys Asp Ser Glu Glu Gly Glu Glu Ser
   180 185 190
   Phe Ser Ser Val Gln Asp Asp Leu Ser Lys Ala Glu Lys Gln Lys Ser
   195 200 205
   Pro Asn Lys Ala Glu Leu Phe Ser Thr Ala Arg Lys Thr Tyr Ser Pro
   210 215 220
   Ala Lys His Gly Lys Gly Arg Ala Ser Glu Asp Ala Lys Gln Pro Cys
   225 230 235 240
   Lys Ser Ala His Arg Lys Glu Ala Cys Ser Ser Pro Lys Ala Ser Ala
   245 250 255
   Lys Leu Ala Leu Met Lys Ala Lys Glu Glu Ser Ser Tyr Asn Glu Thr
   260 265 270
   Glu Leu Leu Ala Ala Arg Arg Lys Glu Ser Ala Thr Glu Pro Lys Gly
   275 280 285
   Glu Lys Thr Thr Pro Lys Lys Thr Lys Val Ser Pro Thr Lys Arg Glu
   290 295 300
   Ser Val Ser Pro Glu Asp Ser Glu Lys Lys Arg Thr Asn Tyr Gln Ala
   305 310 315 320
   Tyr Arg Ser Tyr Leu Asn Arg Glu Gly Pro Lys Ala Leu Gly Ser Lys
   325 330 335
   Glu Ile Pro Lys Gly Ala Glu Asn Cys Leu Glu Gly Leu Thr Phe Val
   340 345 350
   Ile Thr Gly Val Leu Glu Ser Ile Glu Arg Asp Glu Ala Lys Ser Leu
           355                 360                 365
   Ile Glu Arg Tyr Gly Gly Lys Val Thr Gly Asn Val Ser Lys Lys Thr
       370                 375                 380
   Asn Tyr Leu Val Met Gly Arg Asp Ser Gly Gln Ser Lys Ser Asp Lys
   385                 390                 395                 400
   Ala Ala Ala Leu Gly Thr Lys Ile Leu Asp Glu Asp Gly Leu Leu Asp
                   405                 410                 415
   Leu Ile Arg Thr Met Pro Gly Lys Arg Ser Lys Tyr
               420                 425
<210> 4
   <211> 1164
   <212> DNA
   <213> Mus musculus
<220>
   <221> CDS
   <222> (1)..(1164)
<400> 4
   atg gtt gaa gca gat cgc cca gga aag ctc ttc att ggt ggg ctt aat      48
   Met Val Glu Ala Asp Arg Pro Gly Lys Leu Phe Ile Gly Gly Leu Asn
   1               5                   10                  15
   aca gaa aca aat gag aaa gcc ctt gaa gca gta ttt ggc aag tat gga      96
   Thr Glu Thr Asn Glu Lys Ala Leu Glu Ala Val Phe Gly Lys Tyr Gly
               20                  25                  30
   cga ata gtg gaa ata ctt ttg atg aaa gac cgg gaa acc aac aaa tca     144
   Arg Ile Val Glu Ile Leu Leu Met Lys Asp Arg Glu Thr Asn Lys Ser
           35                  40                  45
   aga gga ttt gct ttt gtt acc ttc gaa agc cca gca gat gct aaa gat     192
   Arg Gly Phe Ala Phe Val Thr Phe Glu Ser Pro Ala Asp Ala Lys Asp
       50                  55                  60
   gca gct aga gat atg aat gga aag tcc ttg gat gga aaa gcc atc aag     240
   Ala Ala Arg Asp Met Asn Gly Lys Ser Leu Asp Gly Lys Ala Ile Lys
   65                  70                  75                  80
   gtg gag caa gct acc aaa cca tct ttt gaa agt ggc agg cgt gga cca     288
   Val Glu Gln Ala Thr Lys Pro Ser Phe Glu Ser Gly Arg Arg Gly Pro
                   85                  90                  95
   cct cca cct cca aga agc agg ggc cct ccc aga ggt ctt cga gga gga     336
   Pro Pro Pro Pro Arg Ser Arg Gly Pro Pro Arg Gly Leu Arg Gly Gly
               100                 105                 110
   agt gga gga act agg gga ccc cct tca cgt gga gga tac atg gat gat     384
   Ser Gly Gly Thr Arg Gly Pro Pro Ser Arg Gly Gly Tyr Met Asp Asp
           115                 120                 125
   ggt ggt tat tcc atg aac ttt aac atg agt tct tcc agg gga cca ctt     432
   Gly Gly Tyr Ser Met Asn Phe Asn Met Ser Ser Ser Arg Gly Pro Leu
       130                 135                 140
   cca gta aag aga gga cca cca cca cga agc ggg ggt ccc cct cct aaa      480
   Pro Val Lys Arg Gly Pro Pro Pro Arg Ser Gly Gly Pro Pro Pro Lys
   145                 150                 155                 160
   aga tca aca cct tcc gga cca gtt cga agc agc agt gga atg ggc gga      528
   Arg Ser Thr Pro Ser Gly Pro Val Arg Ser Ser Ser Gly Met Gly Gly
                   165                 170                 175
   aga aca cca gtg tcc cgt gga aga gat agc tac ggg ggt cca cca cga      576
   Arg Thr Pro Val Ser Arg Gly Arg Asp Ser Tyr Gly Gly Pro Pro Arg
               180                 185                 190
   agg gaa ccc ctg cca tct cgc aga gat gtt tat ttg tcc cca aga gat      624
   Arg Glu Pro Leu Pro Ser Arg Arg Asp Val Tyr Leu Ser Pro Arg Asp
           195                 200                 205
   gat gga tat tct aca aaa gat agc tat tca agc aga gat tac cca agc      672
   Asp Gly Tyr Ser Thr Lys Asp Ser Tyr Ser Ser Arg Asp Tyr Pro Ser
       210                 215                 220
   tcc cga gac acc aga gat tat gca ccg cca cca aga gat tat act tac      720
   Ser Arg Asp Thr Arg Asp Tyr Ala Pro Pro Pro Arg Asp Tyr Thr Tyr
   225                 230                 235                 240
   cgt gat tac agt cat tcc agt tcc cgt gat gac tat cca tca aga ggc      768
   Arg Asp Tyr Ser His Ser Ser Ser Arg Asp Asp Tyr Pro Ser Arg Gly
                   245                 250                 255
   tac ggt gat aga gat gga tat ggt cgg gat cgt gac tat tca gat cat      816
   Tyr Gly Asp Arg Asp Gly Tyr Gly Arg Asp Arg Asp Tyr Ser Asp His
               260                 265                 270
   cca agt gga ggt tcc tac aga gat tca tat gag agt tat gga aac tca      864
   Pro Ser Gly Gly Ser Tyr Arg Asp Ser Tyr Glu Ser Tyr Gly Asn Ser
           275                 280                 285
   cgc agt gca ccc cct aca cga ggg cca cca cca tct tat gga gga agc      912
   Arg Ser Ala Pro Pro Thr Arg Gly Pro Pro Pro Ser Tyr Gly Gly Ser
       290                 295                 300
   agt cgc tat gat gat tac agc agc tca cgc gat ggc tac ggt gga agt      960
   Ser Arg Tyr Asp Asp Tyr Ser Ser Ser Arg Asp Gly Tyr Gly Gly Ser
   305                 310                 315                 320
   cga gac agt tac tca agc agc aga agt gat ctc tac tca agt ggc cgt     1008
   Arg Asp Ser Tyr Ser Ser Ser Arg Ser Asp Leu Tyr Ser Ser Gly Arg
                   325                 330                 335
   gat cga gtt ggc aga caa gaa cga ggg ctt ccc cct tct atg gaa agg     1056
   Asp Arg Val Gly Arg Gln Glu Arg Gly Leu Pro Pro Ser Met Glu Arg
               340                 345                 350
   ggg tac cct cct cca cgt gat tcc tac agc agt tca agc cga gga gcg     1104
   Gly Tyr Pro Pro Pro Arg Asp Ser Tyr Ser Ser Ser Ser Arg Gly Ala
           355                 360                 365
   cca aga gga ggt ggc cgt gga ggg agc cga tct gat aga ggg gga ggc     1152
   Pro Arg Gly Gly Gly Arg Gly Gly Ser Arg Ser Asp Arg Gly Gly Gly
       370                 375                 380
   aga agc aga tat                                                     1164
   Arg Ser Arg Tyr
   385
<210> 5
   <211> 388
   <212> PRT
   <213> Mus musculus
<400> 5
   Met Val Glu Ala Asp Arg Pro Gly Lys Leu Phe Ile Gly Gly Leu Asn
   1               5                   10                  15
   Thr Glu Thr Asn Glu Lys Ala Leu Glu Ala Val Phe Gly Lys Tyr Gly
               20                  25                  30
   Arg Ile Val Glu Ile Leu Leu Met Lys Asp Arg Glu Thr Asn Lys Ser
           35                  40                  45
   Arg Gly Phe Ala Phe Val Thr Phe Glu Ser Pro Ala Asp Ala Lys Asp
       50                  55                  60
   Ala Ala Arg Asp Met Asn Gly Lys Ser Leu Asp Gly Lys Ala Ile Lys
   65                  70                  75                  80
   Val Glu Gln Ala Thr Lys Pro Ser Phe Glu Ser Gly Arg Arg Gly Pro
                   85                  90                  95
   Pro Pro Pro Pro Arg Ser Arg Gly Pro Pro Arg Gly Leu Arg Gly Gly
               100                 105                 110
   Ser Gly Gly Thr Arg Gly Pro Pro Ser Arg Gly Gly Tyr Met Asp Asp
           115                 120                 125
   Gly Gly Tyr Ser Met Asn Phe Asn Met Ser Ser Ser Arg Gly Pro Leu
       130                 135                 140
   Pro Val Lys Arg Gly Pro Pro Pro Arg Ser Gly Gly Pro Pro Pro Lys
   145                 150                 155                 160
   Arg Ser Thr Pro Ser Gly Pro Val Arg Ser Ser Ser Gly Met Gly Gly
                   165                 170                 175
   Arg Thr Pro Val Ser Arg Gly Arg Asp Ser Tyr Gly Gly Pro Pro Arg
               180                 185                 190
   Arg Glu Pro Leu Pro Ser Arg Arg Asp Val Tyr Leu Ser Pro Arg Asp
           195                 200                 205
   Asp Gly Tyr Ser Thr Lys Asp Ser Tyr Ser Ser Arg Asp Tyr Pro Ser
       210                 215                 220
   Ser Arg Asp Thr Arg Asp Tyr Ala Pro Pro Pro Arg Asp Tyr Thr Tyr
   225                 230                 235                 240
   Arg Asp Tyr Ser His Ser Ser Ser Arg Asp Asp Tyr Pro Ser Arg Gly
                   245                 250                 255
   Tyr Gly Asp Arg Asp Gly Tyr Gly Arg Asp Arg Asp Tyr Ser Asp His
   260 265 270
   Pro Ser Gly Gly Ser Tyr Arg Asp Ser Tyr Glu Ser Tyr Gly Asn Ser
   275 280 285
   Arg Ser Ala Pro Pro Thr Arg Gly Pro Pro Pro Ser Tyr Gly Gly Ser
   290 295 300
   Ser Arg Tyr Asp Asp Tyr Ser Ser Ser Arg Asp Gly Tyr Gly Gly Ser
   305 310 315 320
   Arg Asp Ser Tyr Ser Ser Ser Arg Ser Asp Leu Tyr Ser Ser Gly Arg
   325 330 335
   Asp Arg Val Gly Arg Gln Glu Arg Gly Leu Pro Pro Ser Met Glu Arg
   340 345 350
   Gly Tyr Pro Pro Pro Arg Asp Ser Tyr Ser Ser Ser Ser Arg Gly Ala
   355 360 365
   Pro Arg Gly Gly Gly Arg Gly Gly Ser Arg Ser Asp Arg Gly Gly Gly
   370 375 380
   Arg Ser Arg Tyr
   385
<210> 6
   <211> 1187
   <212> DNA
   <213> Mus musculus
<220>
   <221> source
   <223> /note="CBA mouse"
<220>
   <221> promoter
   <222> (1)..(1187)
<220>
   <221> TATA_signal
   <222> (1182)..(1187)
<400> 6
   agaaagggga tcagggcagg ctggacagaa cggtgtcata aaaagatgtt ttctttggaa     60
   tgaacctcct atgaggatgt gaaaagacct agaaagggga tcaggggaat gtcagacaca    120
   cgtgtctgtt tcccagacaa gactctgaaa agagagatgg gccacaagtc cctgacacac    180
   ataaggtgac tacttggtcg ctggacccct cacagactgt gtgagtccct ggtctgccaa    240
   ctaggctgcc agaccttgct gggccactgc cacagaagct aggttgctgg ccatcactgt    300
   gtggtgatgg taatggcggg agtatgtgtg tgcacatgct tgtgtgtgca caggtatgaa    360
   agctttcaat ttgccagcaa gggacaggga cagatttggc atacctttaa tatccactgc     420
   ctttcccttc tgtcccagag actggttcct gtgcaggcct ttgcagagtg ctataagaga    480
   atcgagtaag gcttcacttg ttgactgctg ggggctgtga tacctggagg gaagacactg     540
   acccagccta ggggcatcag agctgagagc aggatatcct ggacgcgtga tttgaggaag    600
   gatttcccta gctcactcct gaaggcagtt tcatgaggga tccagaactg gatcatcagc    660
   ccccccctcc ttgaaacaag tgttctcatc ctggggcgct ctgctagcta gatgaccctg    720
   caccaccaac tgccactatc taaaggcagc tattggcctt cctcagactg taggcaaatc    780
   ttgctgctgc cattcgatgc gaagggccag gagtgggtaa actgaggcta aaatggtcca    840
   ggcaagttct gggtgtgtgc gaacgaacca gcggtgggaa cacagagctt ccgggatcaa    900
   agccagacgc cgtccggatt ccggacccag gctcttttcg gggatggttg cctgtgcggc    960
   aggggttggg acgacagtga ccgccagtaa ccccagcgcg cgctggcgca gacgcggtta   1020
   aaggcggacg cccgctagta accccggccc cattcagagc accgggagaa acccgagctg   1080
   ccgccgtcgg gggtgggcgg ggccctaatg gggcgcggcg cggctgctga ttggccatgt   1140
   gcgctcaccc gaggggcggg gcacggaggc gatcggcggg ctttaaa                 1187
<210> 7
   <211> 20
   <212> DNA
   <213> Artificial Sequence
<220>
   <223> oligonucleotide designed to act as primer for amplifying promoter region (ca. 1.2 kb upstream from TATA-like sequence) of SREBP-1c gene.
<400> 7
   gctggacaga acggtgtcat 20
<210> 8
   <211> 26
   <212> DNA
   <213> Artificial Sequence
<220>
   <223> oligonucleotide designed to act as primer for amplifying promoter region (ca. 1.2 kb upstream from TATA-like sequence) of SREBP-1c gene.
<220>
   <221> misc_feature
   <223> oligonucleotide designed to act as primer for amplifying promoter region (ca. 1.2 kb upstream from TATA-like sequence) of SREBP-1c gene.
<400> 8
   taagagctcg gtacctcccc tagggc 26
<210> 9
   <211> 20
   <212> DNA
   <213> Artificial Sequence
<220>
   <223> oligonucleotide corresponding to fructose responsive element in SREBP-1c promoter of CBA/JN Crj mouse.
<400> 9
   ctaaaggcag ctattggcct 20
<210> 10
   <211> 20
   <212> DNA
   <213> Artificial sequence
<220>
   <223> oligonucleotide corresponding to mutated fructose responsive element in SREBP-1c promoter of DBA/2 JN Crj mouse.
<400> 10
   ctaaaggcaa ctattggcct 20
<210> 11
   <211> 24
   <212> DNA
   <213> Artificial sequence
<220>
   <223> oligonucleotide corresponding to sense strand of fructose responsive element in SREBP-1c promoter of CBA/JN Crj mouse.
<400> 11
   aattctaaag gcagctattg gcct 24
<210> 12
   <211> 23
   <212> DNA
   <213> Artificial sequence
<220>
   <223> oligonucleotide corresponding to antisense strand of fructose responsive element in SREBP-1c promoter of CBA/JN Crj mouse.
<400> 12
   aattggccaa tagctgcctt tag 23
<210> 13
   <211> 500
   <212> DNA
   <213> Homo sapiens
<220>
   <221> promoter
   <222> (1)..(500)
<400> 13
   agataatgac tgtcctgttt tctggaggag taaacggagg gttggagcgg ttaaggctcg     60
   ctcagggtgc cagcgaacca gtgatttcga acacagagtt ctggtgtgtt gggccaggac    120
   ttctctgctt tgacccttta acgaaggggg cgggagctga gggccagtga ccgccagtaa    180
   ccccggcaga cgctggcacc gagcgggtta aaggcggacg tccgctagta accccaaccc    240
   cattcagcgc cgcggggtga aactcgagcc cccgccgccg tggggaggtg gggcgggggc    300
   cggggccggg ccctagcgag gcggcagcgc ggccgctgat tggccgcgcg cgctcacccc    360
   atgcccggcc cgcagccccg aagggcgggg cggggcggga cctgcaggcg gggcggggct    420
   ggggcggggc tgggggcggg gcggggcggg gcgggcgcgc cgcagcgctc aacggcttca    480
   aaaatccgcc gcgccttgac                                                 500

## Description

### Field of the Invention

The present invention relates to a novel transcription control cis-element in an SREBP-1c promoter, which is associated with genetic susceptibility to a metabolic disorder, and to a diagnostic method for genetic susceptibility to a sugar or lipid metabolic disorder.

### Background Art

The morbidity rate of type 2 diabetes mellitus has been rising over the past several decades. One important background factor of this trend is that a population in a state called metabolic syndrome, which comprises some metabolic abnormalities such as hyperlipemia, visceral obesity, abnormality of glucose tolerance, and hyperinsulinemia, has recently been increasing. Metabolic syndrome is inducible by environmental factors such as high-calorie diets and a lack of exercise in people with an unidentified genetic background.

Since high-fructose diets induce metabolic disruptions similar to metabolic syndrome in rats, rats loaded with a high-fructose diet are used as an established animal model of metabolic syndrome. Nagai et al. (non-patent document 1) discloses that in rats loaded with a high-fructose diet, the expression of sterol regulatory element binding protein-1 (SREBP-1), a key transcription factor for the expression of a class of lipid synthases in the liver, is induced, whereas the expression of the peroxisome proliferator activated receptor α (PPARα), a ligand-responding nuclear receptor that regulates the expression of a class of enzymes involved in fatty acid oxidation, is downregulated. These changes in the expression of transcription factors may play a central role in the onset of metabolic disruptions in rats loaded with a high-fructose diet.

Furthermore, Nagata et al. (non-patent document 2) discloses that strain-related differences exist in obesity and lipid metabolic abnormalities due to a high-fructose diet in mice, and that these differences are involved in differences in the expression of SREBP-1c in the liver. Specifically, CBA mice had increases in body weight and serum lipid due to a high-fructose diet, whereas DBA/2 mice had no changes; these metabolic abnormalities correlated positively with the expression amount of hepatic SREBP-1c mRNA.

Amemiya-Kudo et al., Journal of Biological Chemistry, Vol. 275(40) pages 31078-31085 (2000) discloses the sequence of the promoter of the mouse SREPB-1c, which contains a guanine residue at the position corresponding to residue 112 of the nucleic acid shown in SEQ ID NO:1.

Deng Xiong et al., Biochemical and Biophysical Research Communications, Vol 290(1), pages 256-262, (2002) discloses sequences of mouse and rat SREPB-1 promoters.

Neither of these documents teaches an SREPB-1c promoter having an adenine residue at the position corresponding to base 112 in SEQ ID NO:1, nor the advantages of such a promoter.

However, the mechanism behind the induction of the expression of the SREBP-1 gene by fructose has not yet been elucidated, nor has been identified a genetic disposition for metabolic abnormalities due to a high-fructose diet.

Non-patent document 1: Nagai et al., "American Journal of Physiology, Endocrinology and Metabolism", (USA), 2002, Vol. 282, No. 5, p. E1180-1190.

Non-patent document 2: Nagata et al., "Tonyobyo (Diabetes Mellitus)", Japan Diabetes Society, April 15, 2002, Vol. 45, Supplementary No. 2, p. S247.

### Summary of the Invention

An object of the present invention is to elucidate the mechanism of induction of the expression of the SREBP-1c gene by fructose in mammals and identify a factor that determines genetic susceptibility to a diet-related metabolic disorder, so as to provide an effective prophylactic or therapeutic agent for a metabolic disorder.

The present inventors conducted diligent investigations aiming at accomplishing the above object, and found that there is a difference in one base in the promoter region of the SREBP-1c gene between CBA mice, which exhibit a lipid metabolic abnormality due to a high-fructose diet, and DBA mice, which exhibit almost no such metabolic abnormality. Furthermore, the inventors conducted a gel shift assay using nucleic acid probes having base sequences comprising the mutated sites in these two groups of mice, and demonstrated the presence of two transcriptional regulatory factors capable of specifically binding only to the base sequence derived from the former strain of mice. As a result of a determination of the amino acid sequences of these transcriptional regulatory factors by TOF-MS analysis, the factors were found to be proteins similar to publicly known Nonamer Binding Protein (NBP) and RNA binding motif protein, X chromosome retrogene (RBMX). The expression of these transcriptional regulatory factors increased remarkably after meals, especially after ingestion of a high-fructose diet, but there was almost no expression during fasting; this expression correlated well with the expression of the SREBP-1c gene.

The present inventors also analyzed publicly known promoters of the human SREBP-1c gene, and confirmed the presence of a sequence homologous to the above-described base sequence comprising the mutated site in CBA mice, and found that a polymorphism similar to that in mice may occur in humans.

The present inventors conducted further investigations based on these findings, and developed the present invention.

Accordingly, the present invention provides:
[1] a nucleic acid comprising the base sequence shown by SEQ ID NO:13, wherein the guanine shown by base number 39 is substituted by adenine;
[2] an ex vivo diagnostic method for genetic susceptibility to a sugar or lipid metabolic disorder in a mouse, which comprises detecting a polymorphism of the base at base number 112 in the base sequence shown by SEQ ID NO:1 in the SREBP-1c promoter in the mouse;
[3] an ex vivo diagnostic method for genetic susceptibility to a sugar or lipid metabolic disorder in a human, which comprises detecting a polymorphism of the base at base number 39 in the base sequence shown by SEQ ID NO:13 in the SREBP-1c promoter in the human.

Because the nucleic acid is capable of detecting the presence or absence of a mutation in the cis-element in the SREBP-1c promoter, it can be used to diagnose genetic susceptibility to a diet-related metabolic disorder in a mammal.

### Brief Description of the Drawings

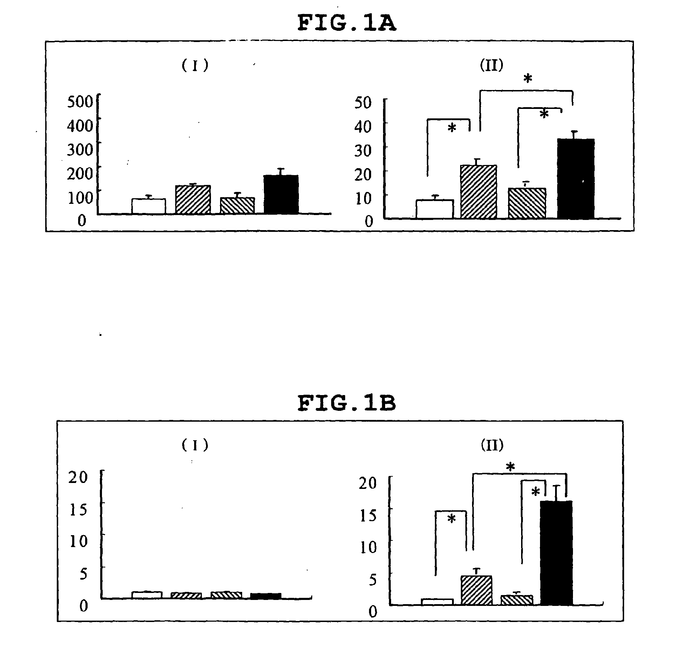

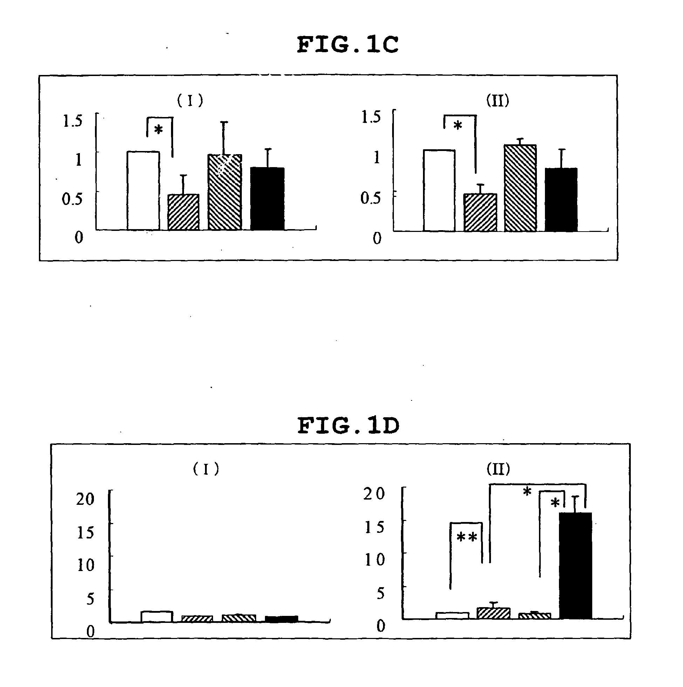

FIG. 1 shows serum TG concentrations (FIG. 1A), SREBP-1c mRNA levels (FIG. 1B), PPAR mRNA levels (FIG. 1C) and FAS mRNA levels (FIG. 1D) in various feeding groups of DBA/2 JN Crj mice (I) and CBA/JN Crj mice (II). In FIG. 1A, FIG. 1B, FIG. 1C and FIG. 1D, the ordinate indicates serum TG concentration (mg/dl), SREBP-1c mRNA level (relative value based on CF group as 1 unit), PPAR mRNA level (relative value based on CF group as 1 unit), and FAS mRNA level (relative value based on CF group as 1 unit), respectively, and the bar graphs show the results from the CF group, the CR group, the FF group, and the FR group, respectively, from left. In these figures, * indicates p< 0.01, and ** indicates p< 0.05.

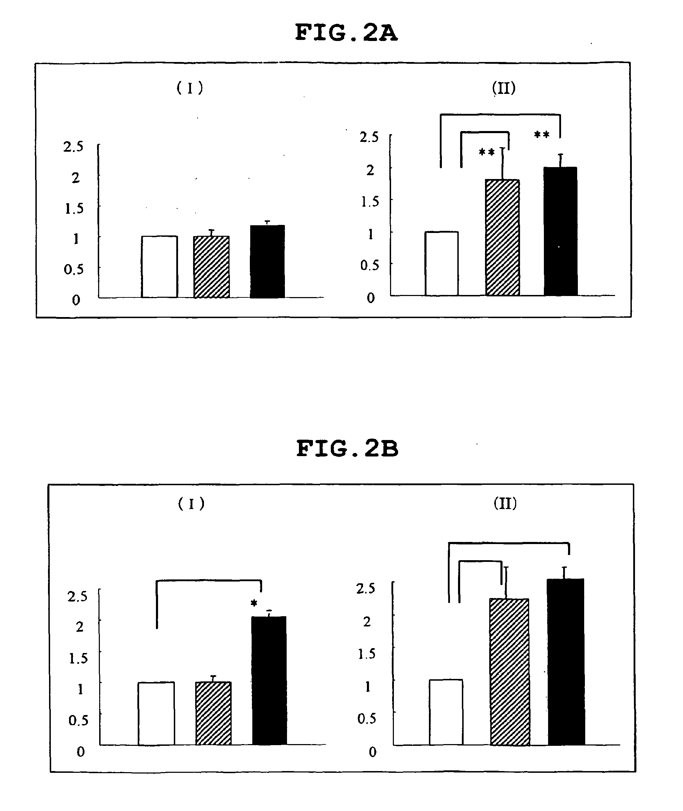

FIG. 2 shows SREBP-1c mRNA levels (FIG. 2A) and FAS mRNA levels (FIG. 2B) in various stimulation groups of primary hepatocytes derived from DBA/2 JN Crj mice (I) and CBA/JN Crj mice (II). In FIG. 2A and FIG. 2B, the ordinate indicates SREBP-1c mRNA level (relative value based on glucose stimulation group as 1 unit) and FAS mRNA level (relative value based on glucose stimulation group as 1 unit), respectively, and the bar graphs show the results from the glucose stimulation group, the fructose stimulation group, and the glucose + insulin stimulation group, respectively, from left. In these figures, * indicates p< 0.01, and ** indicates p< 0.05

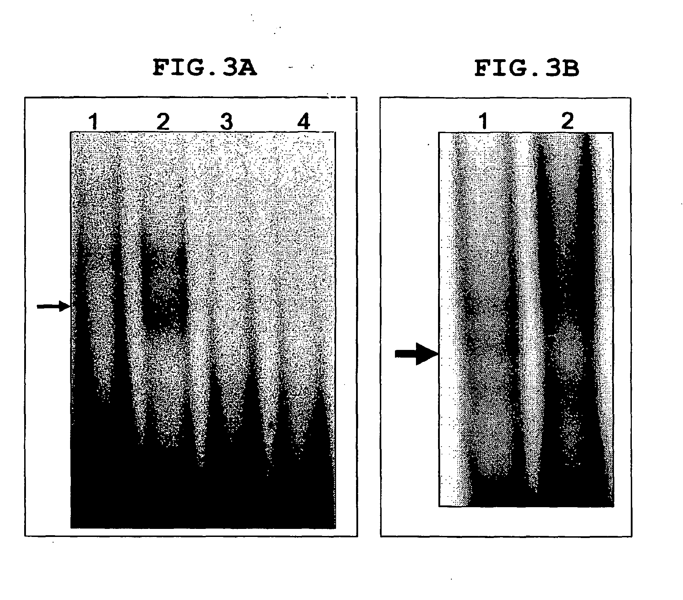

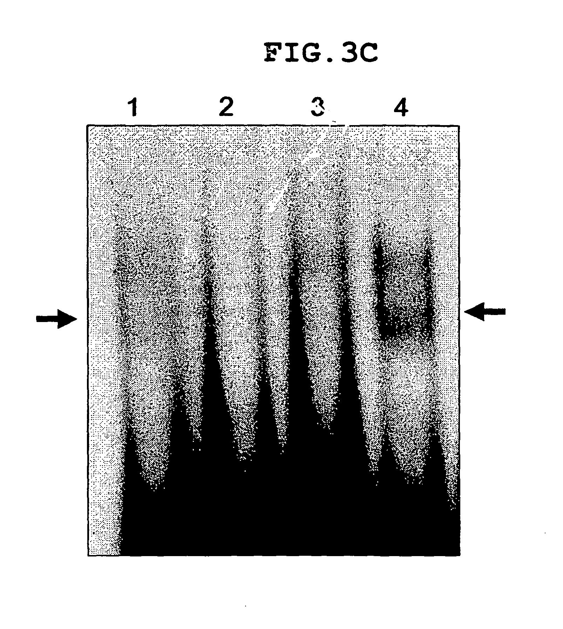

FIG. 3 shows the results of an electrophoretic mobility shift assay (EMSA) for the presence of a transcriptional regulatory factor that binds to the fructose responsive element of the present invention and the regulation of the expression thereof. FIG. 3A shows the results of an EMSA of a nuclear extract derived from CBA/JN Crj mice in the FR group with the CBA probe or the DBA probe (lanes 1 and 2: CBA probe, lanes 3 and 4: DBA probe; lanes 1 and 3: in the presence of cold probe, lanes 2 and 4: in the absence of cold probe). FIG. 3B shows the results of an EMSA of a nuclear extract derived from CBA/JN Crj mice or DBA/2 JN Crj mice in the FR group with the CBA probe (lane 1: nuclear extract derived from CBA/JN Crj mouse; lane 2: nuclear extract derived from DBA/2 JN Crj mice). FIG. 3C shows the results of an EMSA of a nuclear extract derived from CBA/JN Crj mice in the CF group or the FR group with the CBA probe (lanes 1 and 2: CF group, lanes 3 and 4: FR group; lanes 1 and 4: in the absence of cold probe, lanes 2 and 3: in the presence of cold probe).

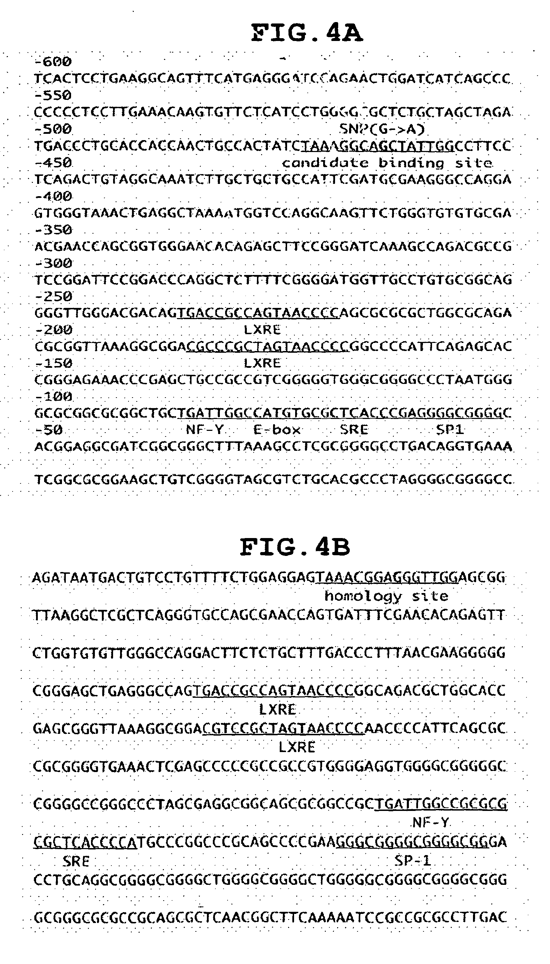

FIG. 4 shows a comparison of the base sequences of the mouse (FIG. 4A) and human (FIG. 4B) SREBP-1c promoters [candidate binding site: candidate binding site of the transcriptional regulatory factor of the present invention (fructose responsive element); SNP (G->A): indicates the occurrence of G→A polymorphism (SNP) in the bold-faced "G"; LXRE: Liver X Receptor (LXR) responsive element; NF-Y: NF-Y binding site; E-box: E-box; SRE: sterol responsive element; SP1: SP1 binding site; homology site: site homologous to the above-described candidate binding site].

### Best Mode for Embodiment of the Invention

The present invention provides nucleic acids which comprise mutants of a transcription control cis-element capable of promoting the transcription of a gene downstream thereof in response to food ingestion (sugar loading), especially to a high-fructose diet (fructose loading) (hereinafter also referred to as "fructose responsive element (FRE) ".

The fructose responsive element (FRE) of an SREBP-1c promoter derived from a mouse of the CBA, C3H or other strain, is shown by SEQ ID NO:1. The base sequence of the human-derived SREBP-1c promoter is shown by SEQ ID NO:13.

The present invention provides mutants SREBP-1c promoter having a mutation in the above-described fructose responsive element, to which a transcriptional regulatory factor capable of binding to the element (that is, the NBP and/or RBMX analogous protein) cannot bind. That is, the mutated FRE of the present invention or the mutant SREBP-1c promoter comprising the mutant FRE is a nucleic acid comprising: the base sequence shown by SEQ-ID NO:1, wherein the guanine shown by base number 112 (G₁₁₂) is substituted by adenine, to which a transcriptional regulatory factor capable of binding to a base sequence consisting of G₁₁₂ and a base adjoining thereto in the base sequence shown by SEQ ID NO:1 cannot bind. The "adjoining base" may be located upstream of the 5' end of G₁₁₂ or downstream of the 3' end, or both. The base sequence consisting of G₁₁₂ and a base adjoining thereto is a base sequence of a total length of about 5 to about 30 bases, preferably consisting of about 5 to about 30 bases, more specifically of G₁₁₂, 0 to 20 bases upstream of the 5' end thereof, and 0 to 20 bases downstream of the 3' of G₁₁₂.

The mutant FRE and a mutant SREBP-1c promoter comprising the same can be used as probes for detecting the mutation in an SREBP-1c promoter of an animal individual, and the mutant SREBP-1c promoter is useful as a transgene and the like for the preparation of a transgenic animal model that is resistant to high-fructose diets.

Alternatively, the mutated FRE of the present invention or a mutant SREBP-1c promoter comprising the same comprises the base sequence shown by SEQ ID NO:13, wherein the guanine shown by base number 39 is substituted by adenine.

The nucleic acid comprising the FRE of the present invention may be any nucleic acid having the above-described base sequence of the FRE of the present invention or a base sequence having one or more bases added upstream of the 5' end and/or downstream of the 3' end of FRE (but excluding nucleic acids comprising the entire base sequence shown by SEQ ID NO:1). The length of base sequence added is not subject to limitation; for example, base sequences comprising an SREBP-1c promoter sequence further upstream of the sequence upstream of the 5' end of G₁₁₂ of the base sequence shown by SEQ ID NO:1 (e.g., base sequence shown by base numbers 1 to 637 in the base sequence shown by SEQ ID NO:6 and the like) are also included.

The nucleic acid may be a DNA, an RNA, or a DNA/RNA chimera, and can be selected as appropriate according to the intended use (e.g., expression promoter, diagnostic probe, therapeutic decoy nucleotide and the like), with preference given to a DNA. The nucleic acid may be single-stranded or double-stranded; in the case of a double-stranded nucleic acid, it may be a hybrid of a DNA strand and an RNA strand. Additionally, the nucleic acid may be physiologically acceptable salts with acid or base. For example, physiologically acceptable acid addition salts are preferred. As such salts, salts with inorganic acids (for example, hydrochloric acid, phosphoric acid, hydrobromic acid, sulfuric acid) or salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like particularly used.

The mutant FRE of the present invention or a mutant SREBP-1c promoter comprising the same can be prepared from a genomic DNA extracted from cells [e.g., hepatocyte, splenocyte, nerve cell, glial cell, pancreatic cell, myelocyte, mesangial cell, Langerhans' cell, epidermal cell, epithelial cell, endothelial cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell or interstitial cell, or a corresponding precursor cell, stem cell or cancer cell thereof, and the like] derived from a human or another mammal (e.g., mouse, rat, rabbit, guinea pig, hamster, bovine, horse, sheep, monkey, dog, cat and the like) having the mutant promoter, for example, from a strain or individual that does not exhibit a metabolic abnormality such as increased serum lipid after meals (especially after ingestion of high-fructose diet), particularly preferably from a DBA or C57BL mouse, or any tissue where such cells are present [e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, salivary gland, peripheral blood, prostate, testicle, ovary, placenta, uterus, bone, cartilage, joint, skeletal muscle, and the like], by cloning a genomic DNA comprising the promoter region with a publicly known SREBP-1c gene promoter sequence (for example, described in Amemiya-Kudo et al., Journal of Biological Chemistry, 2000, Vol. 275, No. 40, p. 31078-31085; GenBank accession number: AB046200) as a probe, cleaving the DNA into a DNA fragment comprising the desired (partial) promoter sequence using a DNA degradation enzyme, for example, an appropriate restriction enzyme, separating the fragment by gel electrophoresis, thereafter recovering the desired band, and purifying the DNA. Alternatively, the mutant FRE of the present invention or a mutant SREBP-1c promoter comprising the same can be isolated by a PCR using a primer synthesized on the basis of a publicly known SREBP-1c gene promoter sequence with a crude extract of the above-described cell or a genomic DNA isolated therefrom as a template.

The mutated FRE of the present invention or a mutant SREBP-1c promoter comprising the same can also be obtained by site-directed mutagenesis by a PCR using a publicly known SREBP-1c gene promoter as a template, with an oligonucleotide having a base sequence having one or more bases (preferably 1 to several bases) substituted, deleted, inserted, or added in the FRE base sequence of the present invention as one primer. Whether the thus-obtained mutant FRE or a mutant SREBP-1c promoter comprising the same does not promote the transcription of a gene downstream thereof in response to food ingestion (sugar loading), especially to a high-fructose diet (fructose loading), can be determined by the binding assay with a transcriptional regulatory factor described below, or by examining changes in the expression of a reporter gene (e.g., luciferase, Green Fluorescent Protein (GFP) and the like) under the control of the mutant promoter due to sugar (e.g., fructose) loading.

Alternatively, the mutated FRE of the present invention or a mutant SREBP-1c promoter comprising the same can also be obtained by chemically synthesizing a base sequence having one base substituted in the FRE base sequence in a publicly known SREBP-1c promoter, using a commercially available DNA/RNA autosynthesizer.

In the case of chemical synthesis, the nucleic acid may be a type of polynucleotide other than deoxyribonucleotide and ribonucleotide, that is an N-glycoside of the purine or pyrimidine base, or another polymer having a non-nucleotide backbone (for example, commercially available protein nucleic acids and synthetic sequence specific nucleic acid polymers) or another polymer having a special bond (however, this polymer comprises a nucleotide having a configuration that allows base pairing or base attachment as found in DNA and RNA) or the like. These may be those having a known modification added thereto, for example, those with a label known in the relevant field, those with a cap, those methylated, those having 1 or more naturally occurring nucleotides substituted by analogues, those modified with an intramolecular nucleotide, for example, those having a non-charge bond (for example, methylphosphonate, phospho triester, phosphoramidate, carbamate and the like), those having a charged bond or a sulfur-containing bond (for example, phosphorothioate, phosphorodithioate and the like), for example, those having a side chain group of a protein (nuclease, nuclease inhibitor, toxin, antibody, signal peptide, poly-L-lysine and the like) or a sugar (for example, monosaccharide and the like), those having an intercalating compound (for example, acridine, psoralen and the like), those containing a chelate compound (for example, metals, radioactive metals, boron, oxidizing metals and the like), or those containing an alkylating agent, or those having a modified bond (for example, -anomer type nucleic acid and the like). Here, "nucleotide" and "nucleic acid" may include not only those containing the purine and pyrimidine bases, but also those containing another modified heterocycle type base. These modified products may contain a methylated purine and pyrimidine, an acylated purine and pyrimidine, or another heterocycle. The modified nucleotide may also have their sugar portion modified by, for example, substitution of one or more hydroxyl groups by a halogen, an aliphatic group (e.g., C₁₋₆ alkyl group) and the like, or conversion to a functional group such as an ether or an amine. As specific examples of the modified nucleic acid, sulfur derivatives and thiophosphate derivatives of nucleic acids, and those resistant to the decomposition like polynucleosideamide or oligonucleosideamide can be mentioned, which, however, are not to be construed as limiting.

Such a modified nucleic acid is useful in increasing in vivo stability and improving cell permeation when used as, for example, a therapeutic decoy nucleotide.

When using a nucleic acid comprising the FRE of the present invention as a gene expression promoter, a base sequence to confer a basal promoter activity, such as the TATA box, is added downstream of the FRE. Furthermore, another transcription control cis-sequence (e.g., CAAT box, GC box and the like) can also be placed at an appropriate position.

The present invention also provides a diagnostic method for genetic susceptibility to a metabolic disorder in a mouse or a human by detecting a mutation in the fructose responsive element in an SREBP-1c promoter. That is, the method comprises detecting a polymorphism of the base at base number 112 in the base sequence shown by SEQ ID NO:1 in the SREBP-1c promoter in the mouse; or detecting a polymorphism of the base at base number 39 in the base sequence shown by SEQ ID NO:13 in the SREBP-1c promoter in the human.

The above-described metabolic disorder include, for example, metabolic disorder due to diet (especially high fructose diet), for example, glucose or lipid metabolic disorder (e.g., hypertriglyceridemia, hyper-LDL-cholesteremia, hypo-HDL-cholesterolemia, obesity, abnormality of glucose tolerance, fasting blood glucose disorder, hyperinsulinemia, hypertension, albuminuria, and the like) and the like.

As a method of detecting a mutation in FRE, any publicly known method of SNP detection can be used. As examples of the detection method, a method wherein hybridization is conducted with accurate control of stringency in accordance with, for example, the method of Wallace et al. (Proc. Natl. Acad. Sci. USA, 80, 278-282 (1983)), using a genomic DNA extracted from cells of a test animal as a sample, with the above-described mutated FRE of the present invention or a nucleic acid comprising the same, as a probe, to detect only a sequence that is completely complementary to the probe, a method wherein hybridization is conducted with gradual reductions in reaction temperature from denaturation temperature using mixed probes prepared by labeling one of the non-mutated FRE described herein or a nucleic acid comprising the same and the mutated FRE of the present invention or a nucleic acid comprising the same, and leaving the other non-labeled, to allow a sequence that is completely complementary to one probe to be hybridized in advance to thereby prevent its cross-reaction with a probe with a mismatch, and the like can be mentioned.

Detection of a mutation in FRE can also be performed by a publicly known PCR-based method of SNP detection, for example, PCR-SSCP method, allele-specific PCR, PCR-SSOP method, DGGE method, RNase protection method, PCR-RFLP method and the like. In the case of the PCR-SSCP method, for example, a PCR is conducted using an SREBP-1c promoter partial sequence upstream of the 5' end of the FRE as a sense primer and an SREBP-1c promoter complementary strand partial sequence downstream of the 3' end of FRE as an antisense primer, with a test animal cell extract or a genomic DNA purified therefrom as a template (one of the primers and substrate nucleotide labeled previously), the resulting amplified fragments are rendered single-stranded and then subjected to non-denatured gel electrophoresis, and primary structural polymorphism can be detected on the basis of the difference in their mobility.

Abbreviations for bases, amino acids and the like used in the present specification and drawings are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His : Histidine
Phe: Phenylalanine
Tyr: Tyrosine
Trp: Tryptophan
Pro: Proline
Asn: Asparagine
Gln : Glutamine
pGlu : Pyroglutamic acid
* : Corresponds to stop codon.
Me: Methyl group
Et: Ethyl group
Bu: Butyl group
Ph: Phenyl group
TC: Thiazolidine-4(R)-carboxamide group

Substituents, protecting groups and reagents frequently mentioned herein are represented by the symbols shown below.
Tos: p-Toluenesulfonyl
CHO: Formyl
Bzl: Benzyl
Cl₂Bzl : 2, 6-Dichlorobenzyl
Bom: Benzyloxymethyl
Z: Benzyloxycarbonyl
Cl-Z : 2-Chlorobenzyloxycarbonyl
Br-Z: 2-Bromobenzyloxycarbonyl
Boc: t-Butoxycarbonyl
DNP: Dinitrophenol
Trt: Trityl
Bum: t-Butoxymethyl
Fmoc: N-9-Fluorenylmethoxycarbonyl
HOBt: 1-Hydroxybenztriazole
HOOBt : 3,4-Dihydro-3-hydroxy-4-oxo-1,2,3-benzotriazine
HONB:1-Hydroxy-5-norbornane-2,3-dicarboximide
DCC: N, N'-Dicyclohexylcarbodiimide

The sequence identification numbers in the sequence listing herein show the following sequences.
[SEQ ID NO:1]
   shows the base sequence of the SREBP-1c promoter region (from immediately before the deduced transcriptional starting site to the upstream of 574^{th} base) derived from CBA strain mouse.
[SEQ ID NO:2] deleted
[SEQ ID NO:3] deleted
[SEQ ID NO:4] deleted
[SEQ ID NO:5] deleted
[SEQ ID NO:6]
   shows the base sequence of the SREBP-1c promoter region (from TATA-like sequence to the upstream of about 1.2 kb) derived from CBA mouse.
[SEQ ID NO:7]
   shows the base sequence of an oligonucleotide designed as a primer for amplifying the SREBP-1c promoter region (from TATA-like sequence to the upstream of about 1.2 kb).
[SEQ ID NO:8]
   shows the base sequence of an oligonucleotide designed as a primer for amplifying the SREBP-1c promoter region (from TATA-like sequence to the upstream of about 1.2 kb).
[SEQ ID NO:9]
   shows the base sequence of an oligonucleotide corresponding to the fructose responsive element in the SREBP-1c promoter from CBA/JN Crj mouse.
[SEQ ID NO:10]
   shows the base sequence of an oligonucleotide corresponding to the mutated fructose responsive element in the SREBP-1c promoter from DBA/2 JN Crj mouse.
[SEQ ID NO:11]
   shows the base sequence of an oligonucleotide corresponding to a sense strand of the fructose responsive element in the SREBP-1c promoter from CBA/JN Crj mouse.
[SEQ ID NO:12]
   shows the base sequence of an oligonucleotide corresponding to an antisense strand of the fructose responsive element in the SREBP-1c promoter from CBA/JN Crj mouse.
[SEQ ID NO:13]
   shows the base sequence of the SREBP-1c promoter region [corresponding to complementary strand sequence of the base sequence represented by base number 16566061-16566560 in the base sequence of human chromosome 17 (accession number: NT_010718) registered in GenBank] derived from human.

The present invention is hereinafter described in more detail by means of the following Examples, which examples, however, are not to be construed as limiting the scope of the present invention.

All materials used were of reagent grade, and were purchased from Nacalai Tesque or Sigma Chemical unless otherwise specified. Numerical data are shown as mean±standard deviation unless otherwise specified. Significant differences among the groups were determined using the Tukey-Welsh tapering multiple comparison. P<0.05 was considered to indicate statistical significance.

### Example 1

### Comparison of metabolic responses of various mouse strains to feeding

Five different inbred strains [BALB/c Cr Slc (purchased from Japan SLC, Inc.); C3H/HeJ (purchased from Charles River Japan); C57BL/6J Jcl, DBA/2N Crj and CBA/JN Crj (all purchased from Clea Japan, Inc.)] of mice (5-weeks old, male) were placed in a animal room with a 12-hour light/dark cycle and allowed to have free access to a laboratory diet and water.

The animals were divided into two groups (an ordinary diet group and a high-fructose diet group) and concurrently fed for 8 weeks. The ordinary diet (Oriental Yeast Co., Ltd.) consisted of 58% carbohydrate (fructose not contained), 12% lipid and 30% protein, and the high-fructose diet (Oriental Yeast Co., Ltd.) consisted of 67% carbohydrate (98% accounted for by fructose), 13% lipid and 20% protein (% values are shown as % calorie).

The day before the experiment, all animals were deprived food at 8:00 p.m. Subsequently, the animals were divided into four groups (4-8 animals per group): 1) animals fed the ordinary diet without refeeding (control-fasted; CF), 2) animals fed the ordinary diet with refeeding (control-refeeding; CR), 3) animals fed the high-fructose diet without refeeding (fructose-fasted; FF), and 4) animals fed the high-fructose diet with refeeding (fructose-refeeding; FR). The mice in the CR group and the FR group were re-fed in the dark between 6:00 a.m. and 8:00 a.m. The CF group and the FF group continued to be fasted. After the body weight (BW) of each mouse was measured, the mouse was anesthetized and laparotomized at 10:00 a.m., the liver and epididymis fat were resected, and the weight of epididymis fat (FW) was measured. Also, various blood tests for blood sugar (BS), triglycerides (TG), total cholesterol (CHO), and insulin (INS) were performed according to conventional methods. The results from the CR group are shown in Table 1, and the results from the FR group are shown in Table 2.

**[Table 1]**

| Mouse | BW | FW | BS | TG | CHO | INS |
|---|---|---|---|---|---|---|
| strain | (g) | (g) | (mg/dl) | (mg/dl) | (mg/dl) | (ng/ml) |
| C3H/HeJ | 23±2 | 0.37±0.11 | 288±31 | 154±12 | 90±7 | 0.3±0.01 |
| C57BL/6J | 23±0 | 0.3±0 | 302±1 | 103±4 4 | 63±1 | 0.1±0 |
| BALB/c Cr SLC | 21±2 | 0.53±0.01 | 269± 7 | 106±15 | 240±7 | 1.3±0.3 |
| CBA/JN Crj | 28±2 | 0.75±0.07 | 252± 8 | 262±35 | 77±2 | 1.2±0.4 |
| DBA/2JN Crj | 23±1 | 0.31±0.06 | 223±22 | 118± 9 | 91±3 | 0.1±0 |

**[Table 2]**

| Mouse | BW | FW | BS | TG | CHO | INS |
|---|---|---|---|---|---|---|
| strain | (g) | (g) | (mg/dl) | (mg/dl) | (mg/dl) | (ng/ml) |
| C3H/HeJ | 33±1^{a} | 1.06±0.12^{b} | 328±30 | 133±16 | 153±16 | 2.3±0^{b} |
| C57BL/6J | 23±0 | 0.5 ±0^{b} | 301±30 | 101±1 | 113± 3^{a} | 0.1±0 |
| BALB/c Cr SLC | 27±1 | 0.77±0.3 | 317± 7^{a} | 153±28 | 316± 7^{a} | 2.4±0^{b} |
| CBA/JN Crj | 31±1 | 1.41±0.12^{a} | 331±19^{a} | 392±43^{a} | 113± 7^{b} | 6.5±0.2^{b} |
| DBA/2JN Crj | 25±1 | 0.5 ±0.13 | 242±15 | 160±27 | 110± 4 | 0.2±0 |

| | | | | | | |
|---|---|---|---|---|---|---|
| a: p<0.05 vs CR; b: p<0.01 vs CR | | | | | | |

In the CBA/JN Crj mice, epididymis fat weight, blood sugar level, TG, CHO, and INS significantly increased with the high-fructose diet compared to the ordinary diet; whereas in DBA/2JN Crj mice, no significant difference was observed between the ordinary diet and the high-fructose diet.

### Example 2

### Correlation between metabolic responses of various mouse strains to feeding and SREBP-1c promoter sequence

After five different inbred strains [C3H/He Slc (purchased from Japan SLC, Inc.); C57BL/6N Jcl, DBA/1JN Crj, DBA/2N Crj and CBA/JN Crj (all purchased from Clea Japan, Inc.)] of mice (5-weeks old, male) were raised using the same feeding method as Example 1 (4-6 animals per group), they were anesthetized at 10:00 a.m. and the livers were resected, immediately frozen in liquid nitrogen and stored at -80°C. Also, serum triglyceride (TG) concentrations were measured according to a conventional method.

Separately, genomic DNA was isolated from each frozen liver using a DNeasy kit (QIEGEN). Primers (sense: 5'-GCTGGACAGAACGGTGTCAT-3'(SEQ ID NO:7) ; antisense: 5'-TAAGAGCTCGGTACCTCCCCTAGGGC-3' (SEQ ID NO:8)) were synthesized on the basis of the publicly known mouse SREBP-1c promoter sequence, and PCR was conducted with each genomic DNA as a template to amplify an about 1.2 kb SREBP-1c promoter fragment. The amplified fragment was subcloned into the TA-Cloning vector (Invitrogen). The base sequence of each insert was determined using the DSQ 1000 fully automated DNA sequencer (Shimadzu Corporation). As a result, it was found that the SREBP-1c promoter of C3H/He Slc and CBA/JN Crj mice had the base sequence shown by SEQ ID NO:6, and that in DBA/2N Crj, DBA/1JN Crj and C57BL/6N Jcl mice, the guanine shown by base number 749 (base number 112 in the base sequence shown by SEQ ID NO:1) (G₁₁₂) has been substituted by adenine in the base sequence shown by SEQ ID NO:6. The relationship between metabolic responses of mice to feeding and the base substitution was examined; in C3H/He Slc and CBA/JN Crj mice, which have a SREBP-1c promoter comprising G₁₁₂, the serum TG concentration rose remarkably after meals (FR group) compared to during fasting (FF group), whereas in DBA/2N Crj, DBA/1JN Crj and C57BL/6N Jcl mice, which have an SREBP-1c promoter with G₁₁₂ substituted by adenine, no significant difference in blood TG concentration was observed between during fasting (FF group) and after meals (FR group) (Table 3).

**[Table 3]**

| Mouse strain | 112-position base | Serum TG concentration (mg/dl) | |
|---|---|---|---|
| | (SEQ ID NO:1) | During fasting (FF) | After meals (FR) |
| C3H/He Slc | G | 80.3±22.5 | 181 ±19.9* |
| CBA/JN Crj | G | 142 ±22.7 | 419 ±17.8* |
| DBA/2N Crj | A | 108 ±63.2 | 126 ±56.2 |
| DBA/1JN Crj | A | 67.5±20.6 | 92.3±26.3 |
| C57BL/6N Jcl | A | 54 ±13.9 | 56.5±10.9 |

| | | | |
|---|---|---|---|
| * : p<0.001 vs FF | | | |

### Example 3

### Differences in lipid metabolism associated gene expression responses to feeding among various mouse strains

After DBA/2N Crj and CBA/JN Crj mice (5-weeks old, male; purchased from Clea Japan, Inc.) were raised using the same feeding method as Example 1 (4-6 animals per group), they were anesthetized at 10:00 a.m. and the livers were resected, immediately frozen in liquid nitrogen and stored at -80°C. Also, serum triglyceride (TG) concentrations were measured according to a conventional method.

Total RNA was isolated from each frozen liver using a TRIzol reagent (GIBCO-BRL Life Technologies, Inc.). The RNA was electrophoresed in a formamide-containing 1% agarose gel and transferred onto a Hybond-N membrane (Amersham Pharmacia Biotech). Probes for SREBP-1, PPAR-α and fatty acid synthase (FAS) mRNA were prepared according to the method described in Am J Physiol Endocrinol Metab 282: E1180-E1190, 2002. The probes were labeled with [α-³²P]dCTP (New England Nuclear Research Products) using a labeling kit (Takara). The membrane was hybridized to each radiolabeled probe in the Perfecthyb Buffer (Toyobo), and washed in 1 x SSC, 0.1% SDS at 68°C for 1 hour. The blot was exposed to a Kodak Biomax MR (Eastman Kodak) film at -80°C. The resulting signals were quantified using a densitometer, and loading differences were standardized against a signal obtained using a probe for 18S ribosome RNA. The results are shown in FIG. 1.

In DBA/2 Crj mice, no significant difference in serum TG concentration was observed among the four groups (CF, CR, FF, FR) ; whereas in CBA/JN Crj mice, the serum TG concentration rose significantly after meals (CR, FR) compared to during fasting (CF, FF) for both the ordinary diet and the high-fructose diet, with greater rises in TG concentration produced by the high-fructose diet (FR) than by the ordinary diet (CR). In starvation, no significant difference was observed between the ordinary diet (CF) and the high-fructose diet (FF) (FIG. 1A). The expression amount of SREBP-1c mRNA correlated well with the serum TG concentration (FIG. 1B). FAS, which is known to undergo expression control by SREBP-1c, exhibited the same expression pattern as SPEBP-1c (FIG. 1D). On the other hand, there was no difference in the expression of PPARα mRNA to control the expression of fat decomposing enzymes between DBA/2 Crj and CBA/JN Crj, with decreased expression observed after meals compared during fasting in both strains of mice (FIG. 1C).

### Example 4

### Differences in lipid metabolism associated gene expression responses of primary hepatocytes to sugar stimulation among various mouse strains

Liver cells were isolated from mice (DBA/2 Crj and CBA/JN Crj (purchased from Clea Japan, Inc.)) in the CF group and the FR group raised in the same manner as Example 1, using the collagenase method with a partial modification. The animals were anesthetized and each liver was perfused with Krebs-Ringer buffer solution (KRB) through the portal vein in situ. Subsequently, the liver was perfused with 100 ml of KRB containing collagenase (Sigma-Aldrich). After the dissociated cells were dispersed with shaking, the resulting dispersion was filtered through a gauge at 4°C in an equal volume of ice cooled DMEM (GIBCO-BRL Life Technology) containing 10% (v/v) fetal calf serum (FCS), 100 µg/ml streptomycin and 100 U/ml penicillin.

The cells were precipitated and twice washed with the same medium at 4°C. An aliquot of 8×10⁶ cells in William's E medium (Sigma-Aldrich) supplemented with 10% (v/v) FCS, 1nM insulin, 100nM triiodothyronine, 100nM dexamethasone, 100 U/ml penicillin and 100 µg/ml streptomycin was seeded onto a 6-well plate coated with rat collagen. After incubation in 9% CO₂ at 37°C for 3 hours, the cells were twice washed with PBS, and incubated using William's E medium supplemented with 1nM insulin, 100nM triiodothyronine, 100nM dexamethasone, 100 U/ml penicillin and 100 µg/ml streptomycin. After incubation for 16 hours, the cells were transferred to William's E medium supplemented with 5mM glucose, 5mM fructose or 5mM glucose +100nM insulin. After incubation for a given time, the cells were recovered, and extraction of total RNA and Northern hybridization using the SREBP-1c or FAS cDNA probe were performed in the same manner as Example 3. The results are shown in FIG. 2.

An in vitro experiment using primary hepatocytes revealed that in CAB/JN Crj mice, stimulation with fructose alone enhanced the expression of SREBP-1c (FIG. 2A) and FAS (FIG. 2B) mRNA to the same level as with insulin stimulation, but in DBA/2 JN Crj mice, no significant difference in the expression of SREBP-1c mRNA was observed among the different stimulations; no difference was observed in the expression of FAS mRNA as well between glucose stimulation and fructose stimulation. Because the expression of FAS mRNA increases with insulin stimulation also in DBA/2 JN Crj mice, it is suggested that a regulatory factor other than SREBP-1c may be involved in the control of FAS expression responses to insulin.

### Example 5

### Identification of a transcriptional regulatory factor that binds to a fructose responsive element in the SREBP-1c promoter

The livers were resected from mice (DBA/2 Crj and CBA/JN Crj (purchased from Clea Japan, Inc.)) in the CF group and the FR group raised in the same manner as Example 1, and a nuclear protein extract from hepatocytes was isolated in accordance with the method of Gorski et al. (Cell 47: 767-776, 1986). The nuclear extract was suspended in 20mM HEPES (pH 7.9), 330 mM NaCl, 1.5 mM MgCl₂, 0.2 mM EDTA, 25% glycerol, 0.5 mM dithiothreitol and 0.2 mM PMSF, and an aliquot of the resulting suspension was frozen in liquid nitrogen and stored at -80°C. Separately, two kinds of radiolabeled double-stranded oligonucleotides comprising the base sequence of G₁₁₂ in the SREBP-1c promoter of CBA/JN Crj mice and in the vicinity thereof (5'-CTAAAGGCAGCTATTGGCCT-3'; SEQ ID NO:9) and the base sequence of the corresponding region in the SREBP-1c promoter of DBA/2 JN Crj mice (5'-CTAAAGGCAACTATTGGCCT-3'; SEQ ID NO:10), respectively (called CBA probe and DBA probe, respectively) were synthesized. An electrophoretic migration shift assay was conducted using these oligonucleotides. 10 µg of nuclear extract, 1 µg of poly (dI-dC), 10mM HEPES (pH 7.9), 60mM KCL, 1mM EDTA, 7% glycerol, and 100,000 cpm labeled probe were mixed and incubated at room temperature for 20 minutes to cause a protein-DNA binding reaction. After incubation, the sample was loaded to 6% polyacrylamide gel in 0.25xTris-borate-EDTA (TBE) buffer, and electrophoresed at a voltage of 150 V. After electrophoresis, the gel was dried and exposed to a film. A competitive assay using a non-labeled oligonucleotide (cold probe) was also performed, and the band that disappeared in the presence of the cold probe was identified as a band of the probe-specifically bound transcriptional regulatory factor. The results are shown in FIG. 3.

The band observed when CBA/JN Crj mouse-derived nuclear extract in the FR group was reacted with the CBA probe (FIG. 3A; arrow) was not observed when the same extract was reacted with the DBA probe. Thus, the presence of a transcriptional regulatory factor that specifically binds to G₁₁₂ in the CBA/JN Crj mouse SREBP-1c promoter and a base sequence in the vicinity thereof was confirmed.

When the DBA/2 JN Crj mouse-derived nuclear extract was reacted with the CBA probe, the band observed when the CBA/JN Crj mouse-derived nuclear extract was reacted with the CBA probe (FIG. 3B; arrow) showed weak signal intensity; it is suggested that the reason why the expression of SREBP-1c does not increase in response to high-fructose diet loading because not only a promoter mutation but also an expression insufficiency of the binding protein may have an effect in DBA/2 JN Crj mice.

Furthermore, when the CBA/JN Crj mouse-derived nuclear extract from the CF group was reacted with the CBA probe, the band observed when the CBA/JN Crj mouse-derived nuclear extract in the FR group is reacted with the CBA probe (FIG. 3C; arrow) exhibited very weak signal, showing a good correlation with the expression of SREBP-1c mRNA in each group. Thus, it was suggested that the SREBP-1c expression response to feed in CBA/JN Crj mice might be controlled by the expression of a transcriptional regulatory factor that binds to the fructose responsive element in the SREBP-1c promoter.

### Example 6

### Amino acid sequencing of a transcriptional regulatory factor that binds to the fructose responsive element in the SREBP-1c promoter

Livers were excised from CBA/JN Crj mice during fasting and at 2 hours after ingestion of a fructose diet, and nuclear protein was extracted in the same manner as Example 5. Two synthetic DNAs (5'-AATTCTAAAGGCAGCTATTGGCCT-3': SEQ ID NO:11; 5'-AATTGGCCAATAGCTGCCTTTAG-3': SEQ ID NO:12) were hybridized to yield a double-stranded DNA comprising G₁₁₂ in the SREBP-1c promoter of CBA/JN Crj mice and a base sequence in the vicinity thereof, and linked to EasyAnchor EcoRI-N (Nippon Gene Co., Ltd., Tokyo) using a TaKaRa ligation kit. 50 µl of EasyAnchor and 200 µg of nuclear protein extract were allowed to stand in the binding buffer used in gel shift analysis at room temperature for 30 minutes. After centrifugation (15,000 rpm, 1 min, 4°C), the precipitate was washed with a washing buffer (100mM KCl, 15mM HEPES-KOH (pH 7.9), 25mM EDTA, 1mM DTT, 0.1mM PMSF, 10% (w/v) glycerol) at 4°C five times, and thereafter the protein was eluted at room temperature with 100 µl of an elution buffer (1.5M KCl, 15mM HEPES-KOH (pH 7.9), 25mM EDTA, 1mM DTT, 0.1mM PMSF, 10% (w/v) glycerol). After centrifugation (15,000 rpm, 1 min, 4°C), the salts in the supernatant were removed by a conventional method, and an electrophoregram was developed by SDS-PAGE. After electrophresis, the gel was stained with silver staining to visualize protein bands; two bands near 41 to 45 kDa that showed a major difference between the sample taken during fasting and the sample taken after ingestion of the fructose diet were cleaved from the gel, and subjected to MALDI-TOF-MS analysis (outsourced to Shimadzu Corporation at Tsukuba) to analyze the primary structures of the proteins. As a result, these proteins proved to be identical to the publicly known Nonamer Binding Protein (GenBank accession number: AAA81558 (SEQ ID NO:3); cDNA was M88489 (SEQ ID NO:2), and to a protein similar to the RNA binding motif protein, X chromosome retrogene (GenBank accession number: AAH11441 (SEQ ID NO:5); cDNA was BC011441 (SEQ ID NO:4).

### Example 7

### Search for a human SREBP-1c promoter sequence homologous to the fructose responsive element in the mouse SREBP-1c promoter

The sREBP-1c promoter sequence of CBA/JN Crj mice and a complementary chain sequence of the base sequence shown by base numbers 16566061-16566560 (SEQ ID NO:13) in the base sequences of human chromosome number 17 (accession number: NT_010718) registered in GenBank, corresponding to the promoter region of the human SREBP-1c gene, were compared using a DNASIS-homology search program. The results are shown in FIG. 4. A sequence homologous to the mouse fructose responsive element ("homology site" in FIG. 4B) was also found in the human promoter.

### Industrial Applicability

The nucleic acid of the present invention, a transcriptional regulatory factor that interacts therewith, and a non-human animal having them transferred or inactivated are not only useful in elucidating the mechanism of induction of a metabolic disorder, but also useful in diagnosing genetic susceptibility to a metabolic disorder, screening for a prophylactic or therapeutic agent for a metabolic disorder and the like.

## Claims

1. A nucleic acid comprising:
the base sequence shown by SEQ ID NO:13, wherein the guanine shown by base number 39 is substituted by adenine.

2. An ex vivo diagnostic method for genetic susceptibility to a sugar or lipid metabolic disorder in a mouse, which comprises detecting a polymorphism of the base at base number 112 in the base sequence shown by SEQ ID NO:1 in the SREBP-1c promoter in the mouse.

3. An ex vivo diagnostic method for genetic susceptibility to a sugar or lipid metabolic disorder in a human, which comprises detecting a polymorphism of the base at base number 39 in the base sequence shown by SEQ ID NO:13 in the SREBP-1c promoter in the human.

## Patentansprüche

1. Nucleinsäure, umfassend:
die in SEQ ID Nr. 13 gezeigte Basensequenz, wobei das Guanin mit der Basennummer 39 durch Adenin ersetzt ist.

2. Ex-vivo-Diagnoseverfahren für eine genetische Prädisposition für eine Zucker- oder Lipidstoffwechselstörung bei einer Maus, umfassend das Nachweisen eines Polymorphismus der Base mit der Basennummer 112 in der in SEQ ID Nr. 1 gezeigten Basensequenz im SREBP-1c-Promotor in der Maus.

3. Ex-vivo-Diagnoseverfahren für eine genetische Prädisposition für eine Zucker- oder Lipidstoffwechselstörung bei einer Maus, umfassend das Nachweisen eines Polymorphismus der Base mit der Basennummer 39 in der in SEQ ID Nr. 13 gezeigten Basensequenz im SREBP-1c-Promotor beim Menschen.

## Revendications

1. Acide nucléique comprenant la séquence de bases présentée en tant que Séquence N° 13, dans laquelle la base guanine n° 39 a été remplacée par une base adénine.

2. Procédé de diagnostic *ex vivo* d'une susceptibilité génétique à un trouble du métabolisme des lipides ou des sucres chez une souris, qui comporte le fait de détecter chez cette souris, dans le promoteur SREBP-1c, un polymorphisme de base au niveau de la base n° 112 de la séquence de bases présentée en tant que Séquence N° 1.

3. Procédé de diagnostic *ex vivo* d'une susceptibilité génétique à un trouble du métabolisme des lipides ou des sucres chez un humain, qui comporte le fait de détecter chez cet humain, dans le promoteur SREBP-1c, un polymorphisme de base au niveau de la base n° 39 de la séquence de bases présentée en tant que Séquence N° 13.
